# EUROPEAN PATENT SPECIFICATION

(11) **EP 1 010 005 B1**
(45) Date of publication and mention of the grant of the patent: **30.07.2008**
(21) Application number: 98943154.9
(22) Date of filing: 07.09.1998
(51) Int. Cl.: G01N 27/416, G01N 33/14

(54) **ELECTRONIC TONGUE**
ELEKTRONISCHE ZUNGE
LANGUE ELECTRONIQUE

(30) Priority: 07.09.1997 SE 9703215
(43) Date of publication of application: 21.06.2000
(73) Proprietor: Senset AB, 583 30 Linköping (SE)
(72) Inventor: WINQUIST, Fredrik, S-584 37 Linköping (SE); WIDE, Peter, S-681 93 Kristinehamn (SE)
(74) Representative: Valea AB
(86) International application number: PCT/SE1998/001583
(87) International publication number: WO 1999/013325

(56) References cited:
- EP-A2- 0 557 642
- GB-A- 2 127 977
- US-A- 4 321 322
- US-A- 4 897 162
- SENSORS AND ACTUATORS B, Volume 44, 1997, ANDREY LEGIN et al., "Tasting of Beverages Using an Electronic Tongue", pages 291-296.
- SENSORS AND ACTUATORS B, Volume 44, 1997, C. DI NATALE et al., "Multicomponent Analysis on Polluted Waters by Means of an Electronic Tongue", pages 423-428.

## Description

There is a growing interest in recent years for the concept of electronic noses. An electronic nose consist of an array of gas sensors with different selectivity patterns, a signal collecting unit and pattern recognition software applied to a computer. The principle is based on the fact that a large number of different compounds contributes to define a measured odor, the chemical sensor array of the electronic nose then provides a pattern output that represents a synthesis of all the components. The pattern output is given by the selectivities of the various sensors.The very essence of the electronic nose is that the combination of several specificity classes has a very large information content.

Electronic noses have already been established for qualitative analysis in various fields of the analytical chemistry, and there are already commercial instruments on the market. Due to the similarities with the human olfaction, these systems have been cited as "electronic noses". Recently, similar concepts, but for analysis in liquids, have been described. These systems are in similar ways related to the tasting sense, thus, for these concepts the terms "electronic tongue" or "taste sensor" have been coined. An electronic tongue was thus described based on a number of chalcogenid glass electrodes, combined with a pattern recognition routine for measurement of metal ions in river water (Ref 1). A more complex sensor composition consisting of glass electrodes and PVC membranes for tasting of beverages has also been described (Ref 3).

Similar concepts, denoted "taste sensors", have also been described. Thus, a LAPS (light addressable potentiometric sensor) with artificial lipid membranes as ion selective material has been described (Ref 4), as well as taste systems, based on a fibreoptical sensor array using potential sensitive dyes (Ref 5) or on a surface photovoltage technique applied to Langmuir-Blodgett films (Ref 6).

A taste sensor based on lipid/polymer membranes on a multichannel electrode has also been developed (Ref 2). This concept has been commercialized as Taste Sensing System SA401, by Anritsu Corp., Japan.

A common feature for these electronic tongues or taste sensors described, that the sensing principle is based solely on potentiometry, the charging of a membrane being measured. This will limit the area of detectable compounds to ions or other charged species.

Thus, these known taste sensors are not sufficiently discriminating to allow for instance quality monitoring in e.g. food processes. The variation in the response to different parameters is not sufficient and despite the use of pattern recognition methods as known from the electronic nose technique the resulting patterns are not separable with sufficient precision.

Furthermore, potentiometric measurements per se are sensitive to electronic noise, putting high demands to the electronics and measurement set-up.

In view of the above mentioned problems encountered with known taste sensors or electronic tongues the object of the invention is to improve the pattern response to variations in tested substances. This object is achieved by a generation of transients by applying electrical pulses to electrodes in contact with the substance to be investigated. For instance voltage pulses may be applied and the current be monitored. Transients will appear at the front and at the rear of the measured current pulse. The transients or some part thereof is then registered and used for comparison purposes, either with measurements at preceding pulses to control continuity in for instance a production process or for comparison what we could call a library of previously registered values for substance detection purposes. For the purpose of simplicity we have in the following mainly discussed the initial transient, however the pulse end transient can be used in a similar manner and at times it may perhaps be of advantage to used measured values from both transients.

Normally in voltametry for instance, the very first moments when electricity is applied to the electrode is not considered at all and one normally waits for the more steady and thus more easily predictable conditions that follow. For the invention which may be employed to give an electronic tongue, it has, however, been found that the initial signal transient response obtained when electricity is applied will vary significantly when the tested substances vary and thus the pattern will also vary with different voltages, pulse wave forms and frequencies.

Contrary to known measuring the initial transients are recorded instead of the later stabilized conditions. The great variation obtained is however of great advantage in the invention (for instance an electronic) tongue were the important thing is to achieve as different patterns as possible for small changes in test substance. Whether the changes in signal response are predictable in a calculable sense of the word or not are however without interest provided that a given change result in the same recognition pattern change every time, which it does. The normal disadvantage can thus be considered as an advantage at the invention.

The use of the transients for recognition (analysis) or monitoring can be done in many different ways. The entire transient curve can be registered and processed or the values at specific times from pulse onset may be used. These values can then be treated by multivariate methods to gain the desired information or control parameters. If these are few only a few significant measured transient values may suffice either consisting of perhaps as little as one value taken after pulse onset but before the peak value, of the peak value only or of a value taken very shortly after the peak, within for instance 90% or preferably 95% of the peak value. Of course also combinations of these can be considered.

In particular when the transient measurements are used together with multivariate methods the resolution or precision may be enhanced by for instance varying the pulse amplitude, for instance the voltage at voltametry. Due to the difference in electric field strength the present substances will react, in particular move, with differing agility depending on size, binding to other substances etc.

In order to also increase the base for multivariate evaluation other parameters the pulse amplitude may be varied as for instance electrode material, pulse frequency, superimposed DC-voltage, catalytic influence and influence from other electrodes. Also different or varying pulse wave forms and/or frequencies, between which a base potential can be applied or superimposed can be considered.

The response signal, for instance the current transient at voltammetry may be measured during the forward step of a square pulse, after return to the initial condition, or both, and if time is in good supply one may also register the equilibrium value between the transients. However one great advantage of the invention is the great speed with which a cascade of identical or differing measurements can be carried out due to the extremely short lived transients, for instance monitoring cycle time can be much shorter than control induced process variations. For the measuring so called "Phase Lock" methods can be used.

The instantaneous faradic current at the electrode is related to surface concentrations and charge transfer rate constants, and exponentially to the difference of the electrode potential from the start value to the final potential. Various parts of the curve thus reflects amount and type of either charged or electroactive species in a complicated and superimposed way.

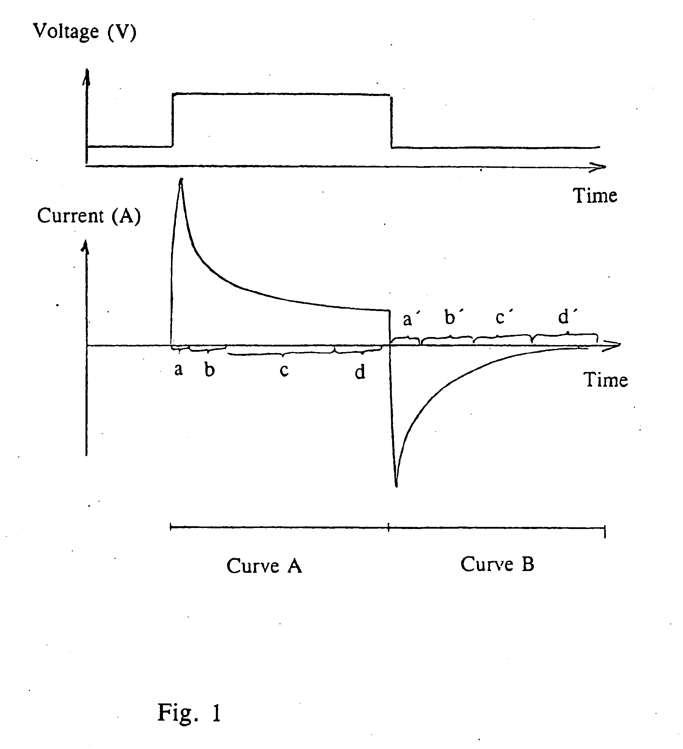

The current - time curve shape (A) due to the onset of a voltage pulse is schematically shown in figure 1.

At least four regions can be distinguished:
a/ Incoming charged and/or redox active species starts to build up a Helmholtz layer. This region is dominated by type and amount of charged species.
b/ Mixed region of incoming charged (dominating part)and/or redox active species
c/ As in b above, but the reaction of redox active species is the dominating part of the current.
d/ Equilibrium is achieved, and the current is solely based on reduction/oxidation of redoxactive species.

Curve B (transient at the end of the pulse) is essentially reversed from that of A. A different shape is, however, obtained due to the different compounds near the electrode surface.

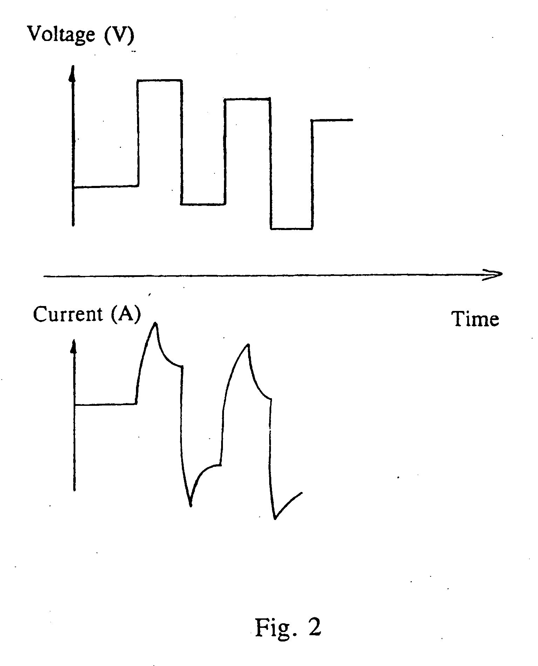

If the voltage pulses are superimposed by a variable signal, for example on a voltage ramp, the corresponding current-time curve will be as shown in figure 2. Similar interpretations as in the previous case can be made.

If in a further development of the invention only or mainly the first part of the transient is measured and used the advantage is gained that a very rapid testing or tasting can be carried out since the first part of the transient is of a very short duration. With the first part is here meant the first rising slope up to the peak. Possibly measurements a short time after this can also be used. However the use of the rising slope of the transient has the advantage of allowing a very rapid pulse frequency resulting in rapid control of for instance food processing. Transients and variations in the control sequences can thus be avoided by using the "taste transients". The achieving of these exceptional results are actually adverse to normal chemical testing since no stable conditions are achieved and instead the chaotic more or less unanalyzable relations are used for the analysis.

Since the pulses may be short the influence of the measuring on the tested substance may become negligible, also the shape and size of the electrodes may be chosen more freely. For instance the electrodes may have a larger size than normally increasing the signal response and an integration over a larger area or volume of the measured substance, diminishing the risk of undesired substance variation influence. Also the short pulses allow rapid testing or monitoring as well as the collection of responses from a great number of pulses with different voltages.

In one configuration of the invention two types of pulse voltammetry may be used, based on large amplitude pulse voltammetry and small amplitude pulse voltammetry, in the following abbreviated as LAPV and SAPV, respectively.

In LAPV, the electrode is held at a base potential, at which negligible electrode reactions occur. After a fixed waiting period, the potential is stepped to a final potential. A current will then flow to the electrode, initially sharp when a Helmholz double layer of charged species is formed and electroactive compounds next to the electrode surface are oxidized or reduced. The current will then decay exponentially as the double layer capacitance is charged and electroactive compounds are consumed, until the diffusion limited faradic current remains. The size and shape of the transient response reflects amount and diffusion coefficients of both electroactive and charged compounds in the solution. When the electrode potential is stepped back to its start value, similar but opposite reactions occur.

In the SAPV, a slow continuous DC scan is applied to the electrode on to which small amplitude voltage pulses are superimposed. This will cause a change in the concentration profile of the electroactive species at the surface. Since only small changes in the electrode potential are considered, this will result in small perturbations in the surface concentration from its original value prior to the application of the small amplitude excitation.

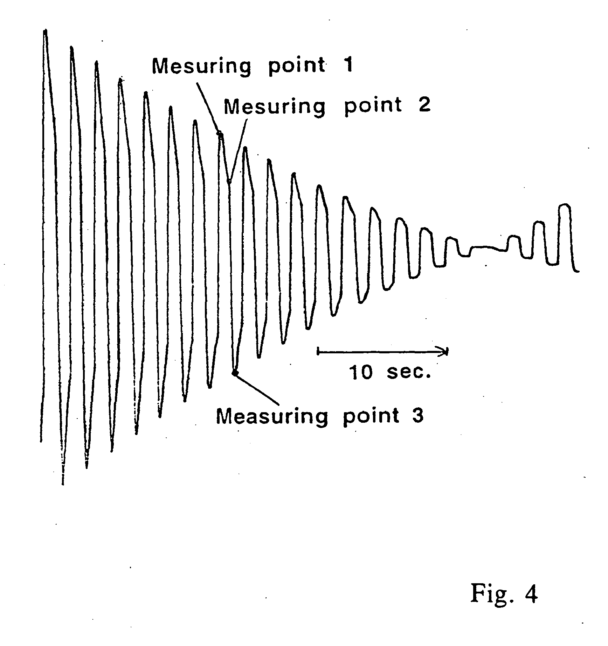

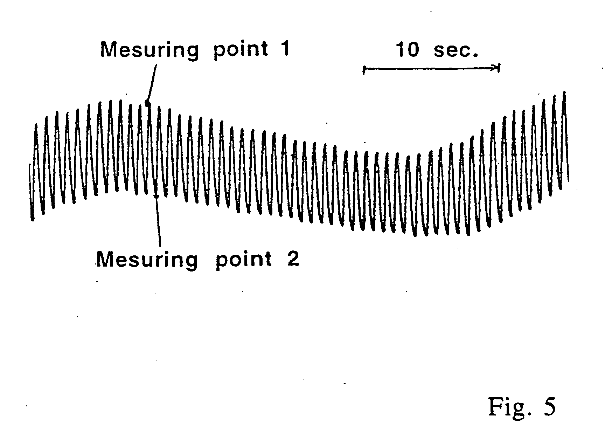

When using pulsed voltammetry, information can also be obtained from AC current versus frequency curves at various potentials. The potential may vary around zero (fig 4) or be superimposed on an other arbitrary statical or dynamical potential curve (fig 5).

In continuous voltammetry, the current depends of the diffusion rate of electroactive species to the working electrode. If the stirring rate in the measurement cell is changed, also the electrode current is changed. One way to overcome this is to use microelectrodes, due to favourable diffusion profiles, an other way is to use pulse voltammetry, conductometry, effect or energy measurements.

Pulse voltametry also enables the use of macroelectrodes that can be cleaned by rather harsh methods, which often is necessary to get clean electrode surfaces. Microelectrode are much more fragile.

The invention also deals with the aspect of influencing the measured solution at one position and to make measurement at an other position, so close that measurement will be affected. This means that compounds generated at one electrode are detected (together with other compounds in the solution) by the other electrode. Since both electrodes may be operated at different potentials and pulse conditions, a very large but also very complicated information concerning the measured solution may be obtained increasing the possible variations in the transients and thus provide a big base for the pattern recognition. In case of streaming or flowing liquids being tested influencing electrodes or materials as for instance catalytic materials can be placed upstream of some electrodes to change the composition before it is tested by other electrodes.

Further developments of the invention are apparent from the subclaims and the following description of experimental tests of the invented method. The description refer to the appended drawings showing:
- Figure 1: A schematic current-time transient due to the on/off set on a voltage pulse;
- Figure 2: A schematic current-time transient due to the on/off set on a voltage pulse, superimposed on a ramped voltage onset;
- Figure 3: Schematics of an experimental set-up;
- Figure 4: A typical recording from LAPV, also showing the position of measurement points. Pulse time and time between pulses are also indicated;
- Figure 5: A typical recording from SAPV, also showing the position of measurement points. Pulse time and time between pulses are also indicated.
- Figure 6: Score plot for the experimental series. The samples were investigated after each other as shown in Table 1.

The basic principle behind the electronic tongue is to combine unspecific and overlapping signal transients with pattern recognition routines. Within this invention, various pulsed voltammetric techniques can be applied to generate information when combined with a multivariate method, such as principal component analysis, partial least square fits, artificial neural nets, fuzzy logic, genetic algorithms or similar statistical or "artificial intelligent" methods. In the invention, also various curve fitting methods may be used to characterize the pulse responses obtained.

The invented method can normally be divided into four steps:
- Use of pulse voltammetry (or other electrical measurement methods) to obtain information (transient curves) ;
- Use of different electrode materials or modified electrodes or pulse voltage etc to obtain different chemical reactions varying the transients;
- Use of curve fitting methods to extract or sample information from the obtained set of transients;
- Use of various multivariate signal processing methods to interpret this thereby gained information.

### Prototype variations of the invention

A prototype of an electronic tongue has been designed, based on the combination of pulse voltammetry using two types of working electrodes and principal component analysis (PCA). This electronic tongue was able to classify various samples, such as fruit juices, still drinks and milk. It was also possible to follow aging processes of milk and orange juice when stored at room temperature.

### Chemicals:

The samples in the experiment consisted of 6 different brands of orange juices.

### Equipment:

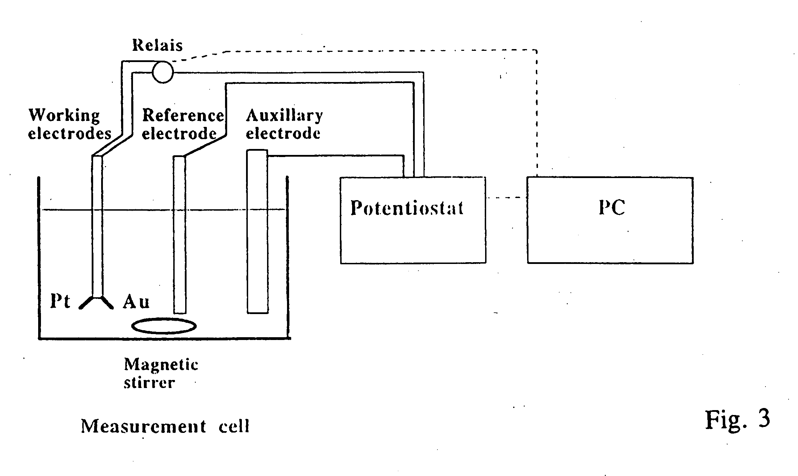

The experiments were carried out in a standard three electrode configuration shown in fig 3, containing a double working electrode 1, an auxiliary electrode 2 consisting of a 20 ***** 50 mm² plate of stainless steel, and a Ag/AgCl (KCl 3M) as reference electrode 3. The double working electrode consisted of one wire of platinum and the other of gold, both with a length of 5 mm and a diameter of 1 mm. The electrode configuration was placed in a 150 ml measurement cell 4, also containing a magnetic stirrer 5 and the cell with content was kept at room temperature. Current transient responses were measured by a potentiostat 6 connected to a PC 7 via an A/D - D/A converter. The PC was used for onset of pulses and measurement of current transient responses and to store data. Via two relays 8, the PC was also used to shift type of working electrode (gold or platinum).

All voltage referred to in the following are versus the Ag/AgCl electrode.

In general for LAPV, a measurement sequence starts by applying a potential during 470 msec., then the voltage is set again to 0 volt during the same time, whereafter the cycle starts all over. By each cycle, the applied potential is decreased by a given value. Measurement values after onset of pulse at 100 msec and 430 msec., respectively, are collected, as well as the measurement value obtained 100 msec. after offset of the pulse, making altogether 3 data points for each cycle. A typical recording is shown in Figure 4, also showing the position of the measuring points. The pulse time, L1, and time between pulses, L2, are also indicated in the figure.

For the SAPV, the potential is scanned from a start value to a final value, and small voltage pulses are superimposed. Each cycle starts by decreasing the potential with a step value during 180 msec., followed by increasing the potential with a superimposed value during 180 msec. Measuring points are collected 100 msec after onset of the step potential and 100 msec after onset of the superimposed potential. The difference between these two measurement points is also taken as a data point, making altogether 3 data points collected for each cycle. A typical recording is shown in Figure 5, also showing the position of the measuring points. The pulse time, L1, and time between pulses, L2, are also shown in the figure.

### Data analysis:

Principal component analysis on the data obtained was performed with a commercial software.

### Results obtained of the prototype:

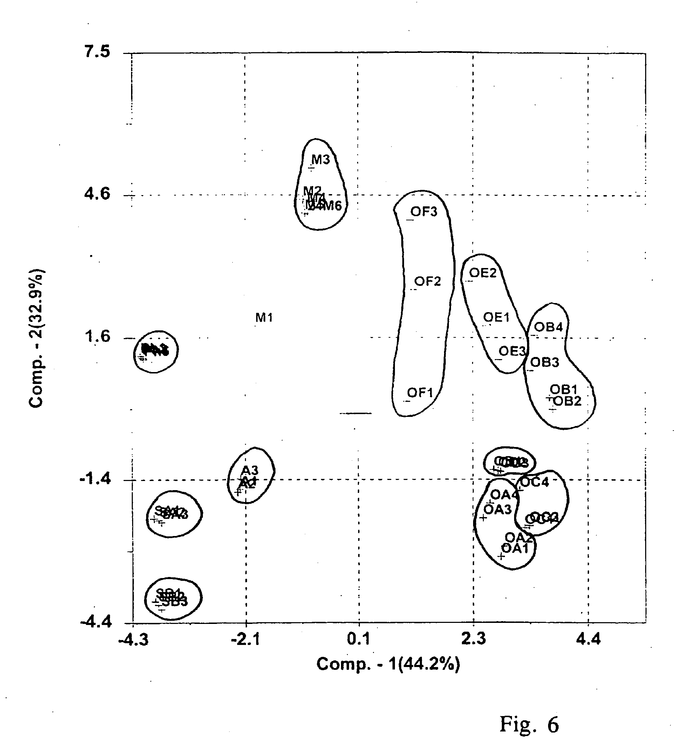

An experimental series were performed, the samples investigated are shown in Table 1. A PCA plot for the whole series of samples is shown in Figure 6. The samples were normalized by dividing by mean in each column. A clear separation between the various samples can be seen.

In the above experiment the concept of an electronic tongue based on the invention demonstrates its capabilities in being able to classify various fruit drinks and milk, and also to be able to follow some aging processes. This opens up future possible applications in e.g. the food industry.

A further development of the concept is also to use other metals as working electrodes, such as palladium, rhodium and iridium or in some other way change the electrode properties by e.g. surface modifications, use of alloys, etc.... Furthermore, electrodes and setups may be used were the measuring electrodes or special electrodes influence the tested liquid. The invention is not limited to liquid matter since solid matters can be tasted by wetting and then testing.

In a practical embodiment of the invention a tasting cell can be a part of a simple pump, for instance a rotary vane pump. The electrodes may be arranged in a wall of the pump body. The axial wall or the peripheral wall. The vane is preferably provided with a brush part that serves as some sort of a seal against the wall. The brush will of course not provide a watertight seal, but since this pump is not intended to give pressure this is not important. What is important is however that the electrodes will be continuously swept clean by the brush. Also the pump or tasting cell will be extremely unsensitive to larger particles. Actually the vane may be constituted entirely of a brush like structure. Alternatively the vane may be a rubber blade pretensioned against the wall of the cell.

Alternatively the pump may comprise a screw in a cylinder, in the wall of which the electrodes are arranged.

The fast measuring or sampling according to the invention allows the use of many electrodes with variations between them as has been described above to give a good base for the use of multivariate recognition or analyzing methods. Together with the above tasting cell two further advantageous possibilities exist.

To start with identical electrodes may be arranged in the feed direction of the pump. If the electrodes consume some molecules in the test sample a reaction decline from electrode to electrode can be measured, or if the electrodes instead leads to an increase in a substance a reaction increase will be detected. This decline or increase may also constitute a part of the recognition pattern.

Secondly the identical electrodes may serve as reserves for each other so that even if one or indeed several become clogged or destroyed the redundant system can continue to measure, but preferably with an indication that all is not well with the cell.

In the case with a screw like brush element centrally in the taste cell, the center of the brush element may constitute auxiliary or reference electrode with the working electrodes on the cylinder wall. The electrodes on the wall may be constituted by circles rendering them rather unsensitive to variations in the measured sample. Also this makes its possible to fabricate a very rugged cell body in a simple way by simply arranging alternating cylindric conductive and insulating rings on top of each other. The electrodes may instead have an axial extension.

Preferably the feeding speed of the pump is kept constant at each test and the in tests that are to be comparable.

If the electrode is not continous around a rotor measuring deviation may occur when the brush passes and shields the electrode. by syncronizing the rotation with the pulse frequency this effect can be made invisible.

**Table 1**

| Sample number | Sample type | Sample number | Sample type | Sample number | Sample type |
|---|---|---|---|---|---|
| 1 | Ph1 | 16 | M4 | 31 | OC4 |
| 2 | Ph2 | 17 | M5 | 32 | OA3 |
| 3 | Ph3 | 18 | M6 | 33 | OA4 |
| 4 | Ph4 | 19 | OE1 | 34 | OB3 |
| 5 | Ph5 | 20 | OE2 | 35 | OB4 |
| 6 | OA1 | 21 | OE3 | 36 | SA1 |
| 7 | OA2 | 22 | OF1 | 37 | SA2 |
| 8 | OB1 | 23 | OF2 | 38 | SA3 |
| 9 | OB2 | 24 | OF3 | 39 | SB1 |
| 10 | OC1 | 25 | OD1 | 40 | SB2 |
| 11 | OC2 | 26 | OD2 | 41 | SB3 |
| 12 | OC3 | 27 | OD3 | | |
| 13 | M1 | 28 | A1 | | |
| 14 | M2 | 29 | A2 | | |
| 15 | M3 | 30 | A3 | | |

Table 1

The experimental series carried out consecutively. The samples are:
Phosphate buffer at pH 7.0: Ph
Orange juice: OA, OB, OC, OD, OE, OF
Milk: M
Apple juice: A
Orange still drink: SA, SB

### References

[1]C. Di Natale, F. Davide, A. D'Amico, A. Legin, A. Rudinitskaya, B.L. Selezenev and Y. Vlasov, "Applications of an electronic tongue to the environmental control", Technical digest of Eurosensors X, Leuven, Belgium (1996) 1345-1348.
[2]K. Toko, "Taste sensor with global selectivity", Materials Science and Engineering C4 (1996) 69-82.
[3]A. Legin, A. Rudinitskaya, Y. Vlasov, C. Di Natale, F. Davide, A. D'Amico, "Tasting of beverages using an electronic tongue based on potentiometric sensor array", Technical digest of Eurosensors X, Leuven, Belgium (1996) 427-430.
[4]Y. Sasaki, Y. Kanai, H. Ushida and T. Katsube, "Higly sensitive taste sensor with a new differential LAPS method", Sensors and Actuators B 24-25 (1995) 819-822.
[5]S. Yamakawa and A. Yamaguchi, "Optical responses of potential-sensitive dye/PMMA coatings to taste solutions", Sensors and Materials 7, 4 (1995) 271-280.
[6]Y. Kanai, M. Shimizu, H. Uchida, H. Nakahara, C.G. Zhou, H. Maekawa and T. Katsube, "Integrated taste sensor using surface photovoltage technique", Sensors and Actuators B 20 (1994) 175-179.

## Claims

1. Measuring method for analyzing a sample substance, comprising the steps of
applying voltage pulses to electrodes in contact with said sample substance to generate a current response transient;
registering said current response transient;
evaluating said current response transient by multivariate signal processing methods.

2. Measuring method according to claim 1, **characterized in** using curve fitting methods to characterize the current response transient obtained to extract or sample information to be evaluated by said multivariate signal processing methods.

3. Measuring method according to claim 1, **characterized in** obtaining at least three data points from the current response transient providing information to be evaluated by said multivariate signal processing methods.

4. Measuring method according to any of the preceding claims, **characterized in** a plurality of voltage pulses being applied consecutively providing a series of current response transients to be evaluated by said multivariate signal processing methods.

5. Measuring method according to claim 4, **characterized in** subsequent voltage pulses being varied to at least one of their amplitude, relation between pulse width and delay between pulses, shape, or frequency.

6. Measuring method according to claim 4, **characterized in** the pulses being superimposed on a rising or falling voltage curve.

7. Measuring method according to any of claims 1 to 6, **characterized in** the use of a plurality of electrodes of different materials.

8. Measuring method according to any of claims 1 to 6, **characterized in** the use of a plurality of electrodes coated by different materials.

9. Apparatus for analyzing a sample substance, **characterized in that** it comprises means for applying voltage pulses to electrodes in contact with said sample substance to generate a current response transient, and
means for registering said current response transient, and
means for evaluating said current response transient by multivariate signal processing methods.

## Patentansprüche

1. Messverfahren zum Analysieren einer Probensubstanz, das die folgenden Schritte aufweist:
Anlegen von Spannungsimpulsen an Elektroden in Kontakt mit der Probensubstanz, um einen Stromantwort-Stoß zu erzeugen,
Aufzeichnen des Stromantwort-Stoßes,
Auswerten des Stromantwort-Stoßes durch multivariate Signalverarbeitungsmethoden.

2. Messverfahren nach Anspruch 1, **gekennzeichnet durch** die Anwendung von Kurvenanpassungsmethoden, um den erhaltenen Stromantwort-Stoß zu charakterisieren, zur Extraktion oder Aufnahme von Informationen, die **durch** die multivariaten Signalverarbeitungsmethoden auszuwerten sind.

3. Messverfahren nach Anspruch 1, **gekennzeichnet durch** Erhalten von mindestens drei Datenpunkten aus dem Stromantwort-Stoß, die Informationen liefern, die durch die multivariaten Signalverarbeitungsmethoden auszuwerten sind.

4. Messverfahren nach einem der vorhergehenden Ansprüche, **dadurch gekennzeichnet, dass** eine Vielzahl von konsekutiv angelegten Spannungsimpulsen eine Serie von Stromantwort-Stößen liefern, die durch die multivariaten Signalverarbeitungsmethoden auszuwerten sind.

5. Messverfahren nach Anspruch 4, **dadurch gekennzeichnet, dass** anschließende Spannungsimpulse in Bezug auf mindestens eine von ihrer Amplitude, ihrer Beziehung zwischen Impulsdauer und Verzögerung zwischen Impulsen, ihrer Form oder ihrer Frequenz verändert werden.

6. Messverfahren nach Anspruch 4, **dadurch gekennzeichnet, dass** die Impulse einer ansteigenden oder abfallenden Spannungskurve überlagert werden.

7. Messverfahren nach einem der Ansprüche 1 bis 6, **gekennzeichnet durch** die Verwendung einer Vielzahl von Elektroden aus unterschiedlichen Materialien.

8. Messverfahren nach einem der Ansprüche 1 bis 6, **gekennzeichnet durch** die Verwendung einer Vielzahl von Elektroden, die mit unterschiedlichen Materialien beschichtet sind.

9. Vorrichtung zum Analysieren einer Probensubstanz, **dadurch gekennzeichnet, dass** sie Folgendes aufweist: Einrichtungen zum Anlegen von Spannungsimpulsen an Elektroden in Kontakt mit der Probensubstanz, um einen Stromantwort-Stoß zu erzeugen; und
Einrichtungen zum Aufzeichnen des Stromantwort-Stoßes; und
Einrichtungen zum Auswerten des Stromantwort-Stoßes durch multivariate Signalverarbeitungsmethoden.

## Revendications

1. Procédé de mesure destiné à l'analyse d'une substance d'échantillon, comprenant les étapes consistant à :
appliquer des impulsions de tension aux électrodes en contact avec ladite substance d'échantillon pour générer un transitoire de réponse de courant,
enregistrer ledit transitoire de réponse de courant,
évaluer ledit transitoire de réponse de courant par des procédés de traitement de signal multivariable.

2. Procédé de mesure selon la revendication 1, **caractérisé par** l'utilisation de procédés d'ajustement de courbe pour caractériser le transitoire de réponse de courant obtenue pour extraire ou échantillonner les informations qui doivent être évaluées par lesdits procédés de traitement de signal multivariable.

3. Procédé de mesure selon la revendication 1, **caractérisé par** l'obtention d'au moins trois points de données du transitoire de réponse de courant fournissant les informations qui doivent être évaluées par lesdits procédés de traitement de signal multivariable.

4. Procédé de mesure selon l'une quelconque des revendications précédentes, **caractérisé par** une pluralité d'impulsions de tension appliquées consécutivement pour fournir une série de transitoires de réponse de courant qui doivent être évaluées par lesdits procédés de traitement de signal multivariable.

5. Procédé de mesure selon la revendication 4, **caractérisé par** la variation des impulsions de tension successives à au moins l'une de leurs relations d'amplitude entre la largeur d'impulsion et le retard entre les impulsions, la forme ou la fréquence.

6. Procédé de mesure selon la revendication 4, **caractérisé par** la superposition des impulsions sur une courbe de tension montante ou descendante.

7. Procédé de mesure selon l'une quelconque des revendications 1 à 6, **caractérisé par** l'utilisation d'une pluralité d'électrodes de différents matériaux.

8. Procédé de mesure selon l'une quelconque des revendications 1 à 6, **caractérisé par** l'utilisation d'une pluralité d'électrodes revêtues de différents matériaux.

9. Dispositif destiné à l'analyse d'une substance d'échantillon, **caractérisé en ce qu'**il comprend un moyen pour l'application d'impulsions de tension aux électrodes en contact avec ladite substance d'échantillon pour générer un transitoire de réponse de courant, et
un moyen pour enregistrer ledit transitoire de réponse de courant, et
un moyen pour évaluer ledit transitoire de réponse de courant par des procédés de traitement de signal multivariable.
